(19) Europäisches Patentamt
European Patent Office
Office européen des brevets

(11) **EP 3 588 428 B1**

(12) **EUROPEAN PATENT SPECIFICATION**

(45) Date of publication and mention
of the grant of the patent:
**07.06.2023 Bulletin 2023/23**

(21) Application number: **18180185.3**

(22) Date of filing: **27.06.2018**

(51) International Patent Classification (IPC):
*G06T 3/40* (2006.01)       *G06T 5/50* (2006.01)
*G06T 5/00* (2006.01)       *A61B 6/00* (2006.01)

(52) Cooperative Patent Classification (CPC):
**G06T 5/50; A61B 6/484; A61B 6/5205;
A61B 6/5282; G06T 5/003;** G06T 2207/10016;
G06T 2207/20221

(54) **PCI CORRECTION**

PCI-KORREKTUR

CORRECTION PCI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.01.2020 Bulletin 2020/01**

(73) Proprietor: **AGFA NV
2640 Mortsel (BE)**

(72) Inventors:
• **NEBOSIS, Rainer
D-81539 München (DE)**
• **LEBLANS, Paul
D-81539 München (DE)**

(74) Representative: **Verbrugghe, Anne Marie L.
Agfa HealthCare NV
IP Department 3802
Septestraat 27
2640 Mortsel (BE)**

(56) References cited:
WO-A1-2017/015381       US-A1- 2017 307 549
US-B2- 9 805 481

• **KREJCI F ET AL: "Single grating method for low
dose 1-D and 2-D phase contrast X-ray imaging",
JOURNAL OF INSTRUMENTATION, INSTITUTE
OF PHYSICS PUBLISHING, BRISTOL, GB, vol. 6,
no. 1, 11 January 2011 (2011-01-11), page C01073,
XP020203529, ISSN: 1748-0221, DOI:
10.1088/1748-0221/6/01/C01073**
• **FABIO DE MARCO ET AL: "Analysis and
correction of bias induced by phase stepping
jitter in grating-based X-ray phase-contrast
imaging", OPTICS EXPRESS, vol. 26, no. 10, 3
May 2018 (2018-05-03), page 12707,
XP055525512, DOI: 10.1364/OE.26.012707**

## Description

## Technical Field

This invention is related to phase contrast imaging, more specifically to a computer-implemented method to reduce specific artefacts that may occur during acquisition and processing. Reference is made in the embodiments to particular examples in medical imaging.

## Background of the invention

Phase-contrast X-ray imaging (PCI) is a general term for different technical methods that use information concerning changes in the phase of an X-ray beam that passes through an object in order to create its images. Standard X-ray imaging techniques like radiography or computed tomography (CT) rely on a decrease of the X-ray beam's intensity (attenuation) when traversing the sample, which can be measured directly with the assistance of an X-ray detector. In PCI however, the beam's phase shift and scattering caused by the sample is not measured directly, but is transformed into variations in intensity, which then can be recorded by the detector.

When applied to samples that consist of atoms with low atomic number Z, PCI is more sensitive to density variations in the sample than conventional transmission-based X-ray imaging. This leads to images with improved soft tissue contrast, hence the particular interest for this technique in medical imaging. Tissue containing a lot of density transitions, such as lung tissue, generates a lot of scatter which makes it highly visible in the scatter image of a phase contrast image.

Conventional X-ray imaging uses the drop in intensity through attenuation caused by an object in the X-ray beam and the radiation is treated as rays like in geometrical optics. But when X-rays pass through an object, not only their amplitude but their phase and propagation direction is altered as well. When applying the values of typical human tissue, the phase-shift of an X-ray beam propagating through tissue is up to three orders of magnitude larger than the loss in intensity caused by the absorption by the same tissue, thus making PCI more sensitive to density variations in soft tissue than absorption imaging.

A further advantage of PCI in medical diagnosis is that, because the phase contrast image formation is not intrinsically linked to the absorption of X-rays in the sample, the absorbed dose can potentially be reduced by using higher X-ray energies potentially saving the patient from substantial dose depositions during X-ray image acquisition.

Phase differences in X-ray beams cannot be directly measured by an X-ray detector, but require special interferometry techniques using diffraction gratings or an in-coherent setup with a plurality of apertures to produce the measurable effects at the X-ray detector. These measurable effects are small variations in the direction and width of X-ray beamlets obtained e.g. by means of a grating in between an X-ray source and a subject under examination, said variations being detected by means of an assembly of a detector mask placed before an X-ray detector.

Basically two approaches exist to produce said measurable effects:

1. An interferometric approach which uses a partially coherent source of radiation referred to as Talbot-Lau interferometry.

2. A completely in-coherent approach with repeated edge illumination referred to as Coded Aperture Phase Contrast Imaging. The present invention is applicable to the second approach and will be described further on with reference to the Coded Aperture Phase Contrast imaging technique. The present invention is not limited to this embodiment. The principle of coded aperture phase contrast imaging (PCI) has been described in US 7, 869, 567.

For Coded Aperture Phase Contrast Imaging to work, X-rays emitted from an X-ray source are shaped into individual beamlets by means of selective absorption by a so-called "pre-sample mask" or "coded aperture" (which is an absorbing grating). The pre-sample mask allows only narrow portions of the X-ray beam to pass through so-called apertures of identical width. This pre-sample mask traditionally has a fixed geometry that is adapted to an analyser grating that in its turn is fitted to the geometry of the used X-ray detector pixel grid dimensions while taking into account the distances between the X-ray source, pre-sample mask and X-ray detector. The analyser grating, also called "detector grating", blocks a part of the beamlets dependent on the relative position of the beamlet and the analyzer grating. The pitch of the beamlet structure (determining the X-ray intensity profile) and the analyzer grating are adapted to the pitch of the detector.

These narrow beamlets pass through a sample or object and arrive at individual pixels of the X-ray detector through said analyser grating. The individual X-ray beamlets are arranged to hit the pixel edge of individual rows of pixels or individual columns of pixels or individual pixels. Small deviations in the individual beamlets cause a significant increase or decrease in the signal hitting the exposed area of the pixel resulting in a significant phase contrast or dark field signal.

At the same time, the physical analyser grating blocks by definition a considerable portion of the radiation passing the imaged object or patient, and that does not contribute to the image acquisition. The physical analyser grating intrinsically reduces the yield of the applied radiation dose.

In an earlier filed patent application (EP17181154.0) by the same applicant, a solution has been proposed to replace a physical analyser grating by a so-called virtual grating. The virtual grating is based on the principle that rather than physically partially blocking the incidence of a beamlet for a selected detector pixel rows, the detector pixel rows in question are switched off for detection (or the pixel rows in question are not considered for image reconstruction). This principle thus allows to capture the entire detector area and not causing loss of incident dose usage. The detector pixel rows that are to be blocked for acquiring the phase contrast data are selected after the actual image acquisition. This improvement reduces the number of required exposures to obtain a full resolution phase contrast image, and thus reduces the applied dose to the patient.

As suggested above, there exist two types of measurable effects caused by said aforementioned incidence of X-ray beamlets on an object and causing said small deviations.

The first effect is the pure deflection of the X-ray beamlets influenced by the shape of the object or large structures inside the object (large in this context means some 100 $\mu$m in diameter) and giving rise to the "phase image".

Secondly, scattering of the X-ray beamlets passing through an object and influenced by the path-length of the beam through the object -and thus by the size and internal small structures (small in this context means below 100 $\mu$m in diameter) of the object- giving rise to the "scatter image" or "dark field image".

Typically, the re-calculation of the phase image is based on the difference of two images recorded at different positions of the analyser grating (or different sets of pixels for the approach of virtual analyser grating). The scatter image typically uses the sum of two normalized images, as determined by the following equation system:

$$
\begin{cases}
t = \dfrac{2x_1}{A_{MN}} \sqrt{\dfrac{\pi}{D+C}} I_2 \exp\left[\dfrac{1}{2^4}\dfrac{(D-C)^2}{D+C}\right] \\[3mm]
\Delta x_R = \dfrac{x_1}{2}\dfrac{D-C}{D+C} \\[3mm]
\sigma_M^2 = \dfrac{2x_1^2}{D+C} - \sigma_N^2,
\end{cases}
$$

where $C = -2\ln(I_1/I_2)$ and $D = -2\ln(I_3/I_2)$.

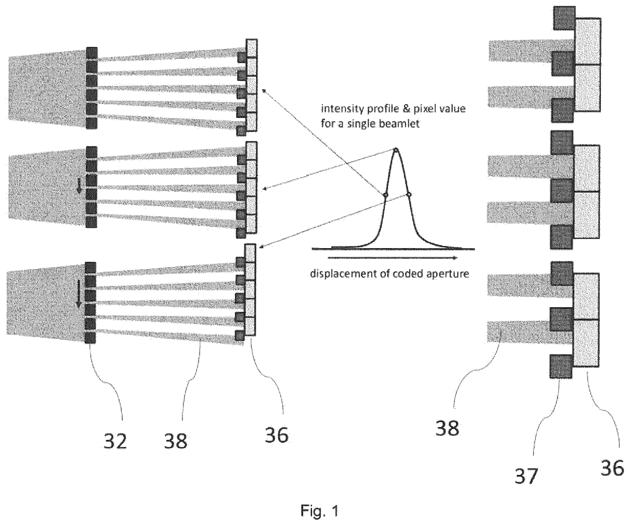

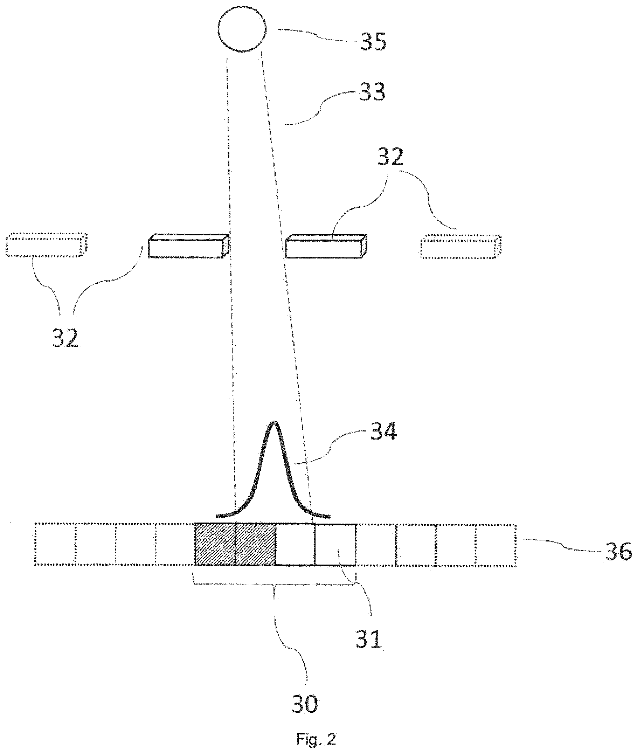

In the above equation, the classical absorption x-ray image is denoted as t; $\Delta x_R$ is the phase image and $\sigma_M^2$ the dark field image. Visualization of the effects of beam deflections on the recalculating of the phase images and the scatter images are depicted in Fig.1 and Fig. 2 respectively.

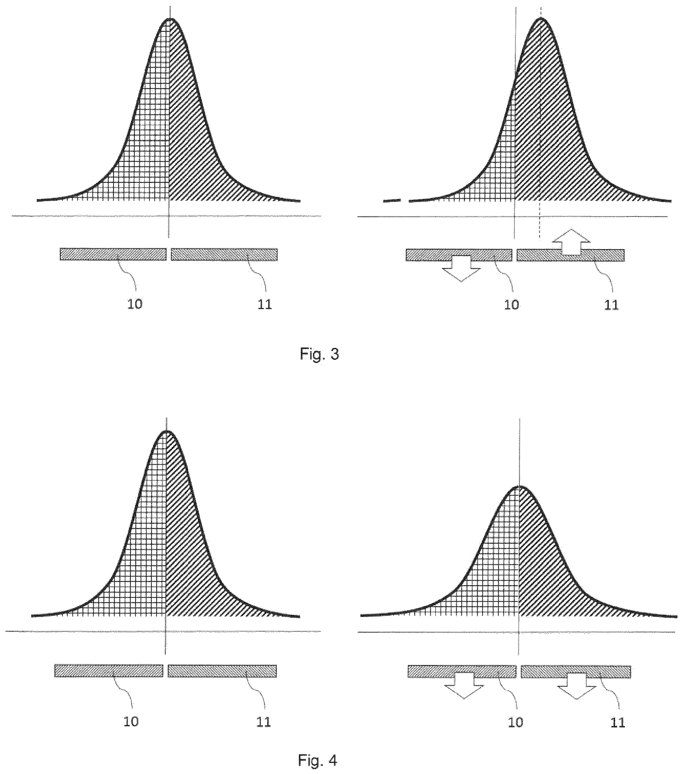

In Fig. 3 the original situation is depicted on the left side wherein the doses recorded in 2 neighbouring pixels (10) and (11) are equal in the case of an undisturbed X-ray beam (i.e. the X-ray beamlet is not influenced by any deflection by the imaged object or patient). The situation on the right side of Fig. 3 represents the case where there is beam deflection resulting in a shift to the right of the centre of the beamlet. In this case, pixel (11) will record an increased dose at the expense of a decreased dose recorded in pixel (10). The difference between pixel (10) and (11) can be understood as a measure for the phase change.

Fig. 4 shows the effect of beam broadening by scatter. The beam gets broader and lower (scatter leaves the area under the curve unchanged - no absorption). Due to beam broadening a part of the intensity is deflected to the neighbouring pixels. The dose captured in both pixels (10) and (11) goes down by the same amount.

Now the problem to be solved becomes obvious: if the imaged object is absorbing part of the incident radiation, this effect will also lead to a decrease of the doses recorded by both pixels (10) and (11). To discriminate between a dose reduction due to absorption or to scatter effects, it is necessary to normalize the pixel values in some way. In the situations known in the state of the art, this is typically achieved by recording a third image with the pixels centred to the intensity profile. This approach however implies that an additional acquisition has to be made (with an effect of increased dose for the patient).

Moreover, even when an additional exposure is made to allow the normalisation, this approach will not work under certain circumstances. In the case that a strong absorption gradient is present in the imaged object, this causes a difference between the readings of the first (10) and second pixel (11) even without a phase gradient and thus mimics a beam deflection.

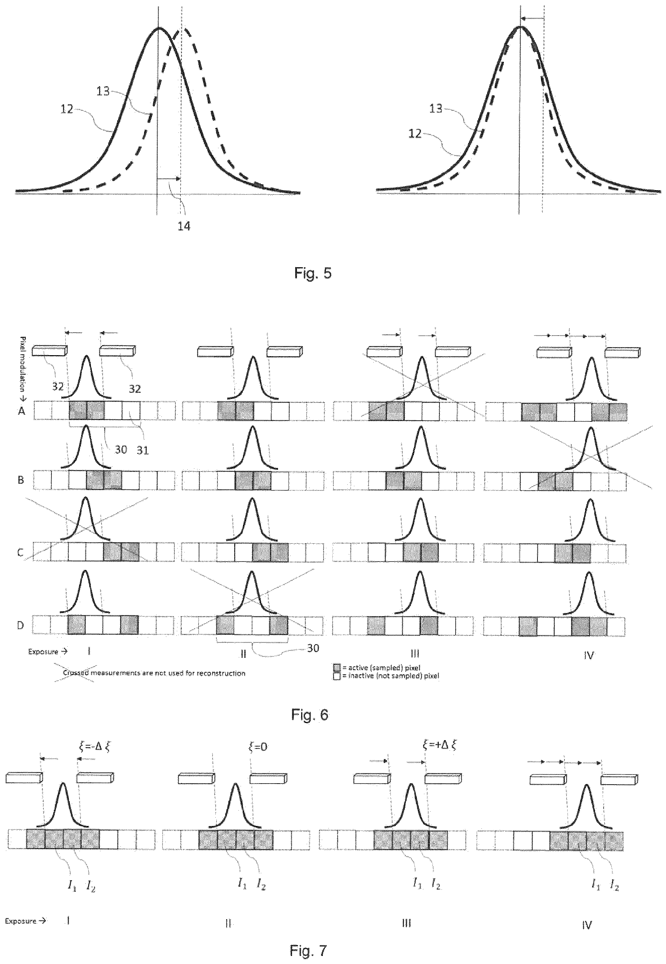

The impact of an absorption gradient can be illustrated by means of a simple graphical simulation (refer to the left graph in Fig. 5). A Gaussian profile (12) was multiplied with a linear function which represents the absorption gradient. The result of such a superposition (13) is that the centre of the profile shifts with increasing gradient to the right (14). Using standard re-calculation would lead to a wrong phase image. Moreover the absorption gradient slightly decreases the width of the profile. With standard re-calculation this would be interpreted as negative scattering coefficient which is physically impossible.

The effects of a steep absorption gradient have an impact in both the phase image and the scatter image. In phase image, the profile shifts off-centre. The phase effect therefore is overestimated (and therefore wrong). In the scatter image, the profile becomes steeper, i.e. the scatter effect is underestimated.

To solve this problem, typically additional images acquired with different positions of the pre-sampling mask and the analyser grating are recorded. To calculate the uncorrected phase image at least two images at different positions of the analyser grating are needed. To correct for absorption gradients 4 additional images are recorded at 2 different positions of the pre-sampling mask and for each position of the pre-sampling mask 2 images with different positions of the analyser grating.

These 4 additional images are only used to calculate the absorption gradient. This increases recording time and dose. Moreover 2 motorized stages are needed: one to move the pre-sampling mask the other to move the analyser grating.

In summary, the state of the art approach to compensate for the effects of a steep absorption gradient leads to three disadvantages: longer recording time, high dose (to patient) and a higher complexity of the system.

For the sake of completeness it has to be mentioned that there are phase contrast imaging system designs which produce only very small artefacts in the presence of an absorption gradient. These designs however require mini-focal-spot x-ray tubes (spot size <100 $\mu$m) and very fine grating structures (pitch < 100 $\mu$m). Such systems only have very little chances to fulfil the requirements for clinical applications. Their X-ray flux is much too low to make an X-ray image with acceptable quantum noise in an acceptable time. Their grids are very hard to produce and will be much too costly for a medical X-ray system with an acceptable price. An X-ray sensor is needed with pixels that are smaller than the pixels of current medical flat-panel detectors.

KREJCI F ET AL: "Single grating method for low dose 1-D and 2-D phase contrast X-ray imaging", JOURNAL OF INSTRUMENTATION, INSTITUTE OF PHYSICS PUBLISHING, BRISTOL, GB, vol. 6, no. I, discloses an X-ray phase contrast imaging method wherein a single grating is used to obtain phase gradient information and the corresponding absorption image from a single X-ray exposure. To that purpose, a specific absorption grating is used that casts an X-ray shadow on an X-ray detector of which regions $I_1$, $I_2$, $I_3$ and $I_4$ contribute to said phase gradient information. The method however does not rely on the capturing of multiple images corresponding to different positions of the pre-sample mask, correcting absorption gradient artefacts in said images and combining the corrected images to obtain a high-resolution PCI image.

Patent applications US 2017/307549 A1 and WO 2017/015381 A1 both disclose methods removing the analyser grating (often known as G2) and including it in the image detector, so that from one-exposure data, $n$ PCI images are determined by using the virtual matrix overlay on the detector.

FABIO DE MARCO ET AL: "Analysis and correction of bias induced by phase stepping jitter in grating-based X-ray phase-contrast imaging", OPTICS EXPRESS, vol. 26, no. 10, 3 May 2018, discloses a grating-based x-ray phase contrast imaging method that detects and removes artefacts due to processing of phase-stepping data with incorrect stepping positions, such as spatial sampling jitter. The method discloses that in order to obtain a baseline reference, the phase-stepping procedure is performed twice: once with and once without the imaged object, but it does not disclose that the corrected phase and scatter profile measurements are determined by normalization with neighboring profiles.

US2017213364A discloses a method to reconstruct under-sampled PCT data by performing a regularized Fourier analysis on the under-sampled scan data and correcting for one or more PCT system contributions to the under-sampled scan data by dividing the computed Fourier coefficients by Fourier coefficients representative of the one or more PCT system contributions, obtaining at least one of an absorption sinogram, a differential phase sinogram, and a dark field sinogram from the corrected Fourier coefficients, and performing tomographic reconstruction on the obtained absorption sinogram, the obtained differential phase sinogram, and the obtained dark field sinogram.

## Summary of invention

The present invention provides a method as set out in Claim 1.

In the context of this invention, a high-resolution PCI image is either a phase image or a dark field image that has been acquired at a high spatial resolution, for instance at the native resolution of conventional X-ray detector, wherein each detector pixel corresponds to an image pixel in the resulting PCI image. The method described here is based on the principle that while the reconstruction of a single pixel of a PCI image requires a profile measurement of a set of ($n$) neighbouring pixels (and thus intrinsically decreases the spatial resolution of the resulting PCI image by a factor $n$), this loss in spatial resolution can be compensated by acquiring additional acquisitions of $n$ x-ray absorption images.

When a pre-sample mask or beam splitter is used in the radiation path from X-ray source to detector some detector pixels of the detector are not irradiated. Hence the total pixel resolution of the detector is not used. In order to increase the resolution again, the pre-sample mask in a specific embodiment is shifted in a direction parallel to the plane of the detector and so that pixels of the pixel matrix which were not irradiated are now available and can be used to calculate a second, third etc. set of absorption, phase images and dark field images by applying the method described higher. This shifting of the pre-sample mask is done at most n-1 times in order to obtain *n* x-ray absorption images in total to leverage the full spatial resolution of the digital image detector. These additional acquisitions need to fulfil certain criteria matching the geometry of the PCI system.

In the context of this invention, the PCI system to which the method may be applied comprises an X-ray source, a pre-sample mask (or beam splitter) and a digital imaging detector. The PCI system of this invention does not have a physical analyser grating or detector mask, which - although being an essential component of a coded aperture PCI system- is replaced by a so-called virtual analyser grating or virtual grating, as disclosed in patent application (EP17181154.0). This virtual grating replaces the presence and function of a physical analyser grating by selectively reading out predetermined sequences of detector pixel groups of the same image, rather than physically blocking incident radiation by grating bars.

The virtual grating is implemented as a virtual mask laid over the pixel matrix of the recorded pixel values and thereby "blocking" or "ignoring" some pixels so that their values are not used in the computation of the absorption, phase or dark field image while others remain available for use in the computation. By shifting the virtual mask so that pixels that were used in the computation of one of the above-mentioned images become blocked while other sets of (neighbouring) pixels become available for computation of one of these images. The virtual grid thus attains the same result as obtained by the moving analyser grating or detector mask in the state of the art.

In the context of this invention, absorption gradient artefacts are image artefacts in the phase image or the dark field image which are caused by the presence in the imaged object of a steep gradient in electron density in the imaged object. These steep gradients typically occur at transitions between tissues of different densities (e.g. between tissue and air). The consequence is that in the phase image, the phase effect is overestimated (the profile shifts off-centre), and the dark field image the scatter effect is underestimated (as the profile becomes more steep).

An absorption image is a conventional X-ray absorption image.

In the context of the invention, an uncorrected image relates to whether or not a PCI image has been corrected for the absorption gradient artefacts.

In the context of this invention, normalization of a measured profile means that the maximum value of the profile (that is to be normalized) is mathematically compared with the maxima of at least two neighbouring measured profiles, and then adjusted to the mean of the calculated maxima. The neighbouring profiles are defined as the profiles of which the centres coincide with the neighbouring pixels of the centre of the profile to be normalized. These neighbouring profiles are thus acquired by physically offsetting the pre-sample mask to directly neighbouring presample mask positions, with a distance so that the projection at the level of the detector corresponds to the width of a detector pixel.

Normalization may also be achieved by subtracting the pixel values;

$$ph = ph_{nc}\big(I_1|_{\xi=0};\ I_2|_{\xi=0}\big) - c\big(I_1|_{\xi=-\Delta\xi};\ I_2|_{\xi=-\Delta\xi};\ I_1|_{\xi=+\Delta\xi};\ I_2|_{\xi=+\Delta\xi}\big)$$

or

$$ph = ph_{nc}\big(I_1|_{\xi=0};\ I_2|_{\xi=0}\big)/c\big(I_1|_{\xi=-\Delta\xi};\ I_2|_{\xi=-\Delta\xi};\ I_1|_{\xi=+\Delta\xi};\ I_2|_{\xi=+\Delta\xi}\big)$$

Normalization in general means to divide a set of values by a number to either make the maximum equal to one or the integral of the data set equal to one. In case of X-ray absorption the intensity follows an exponential decay: $I \sim e^{-\mu(x)\cdot z}$ with $\mu(x)$ being the local mean absorption coefficient along the path z through the object. Dependent on the type of normalization (normalizing by the local intensity variation or the local absorption coefficient variation) the normalization is a division or subtraction as $\mu \sim ln(I)$ and $ln\left(\frac{a}{b}\right) = ln(a) - ln(b)$. In the specific case of the context of this invention the normalization procedure depends on whether the intensity impinging on the detector pixel or the logarithm of this value is used to calculate the correction term.

Specific examples and preferred embodiments are set out in the dependent claims.

The present invention can be implemented as a computer program product adapted to carry out the steps as set out in the description. The computer executable program code adapted to carry out the steps set out in the description can be stored on a computer readable medium.

The present invention is an improvement to the technique referenced above in EP17181 154.0 and reduces a number of very specific image artefacts which may occur due to the presence of steep X-ray absorption gradients in the imaged objects. The invention is advantageous in that the method allows for reducing artefacts in the PCI image which are caused by steep absorption gradients in the imaged object without the need to acquire additional images for this purpose.

Further advantages and embodiments of the present invention will become apparent from the following description and drawings.

**Brief description of drawings**

Fig. 1 is a schematic diagram explaining the principle of a conventional PCI image acquisition. In order to be able to acquire and reconstruct a phase and dark field image, a series of absorption images have to be taken. The left side of the drawing shows three series of X-ray beams (top to bottom) being split by a pre-sample mask (32). The pre-sample mask is in three different positions for the subsequent acquisitions (the arrow indicates the pre-sample mask being moved). The split beam (38) falls on a digital image detector of which the individual detector pixels (36) are partially blocked by a physical detector grating (37).

Fig. 2 is a schematic diagram of a PCI system of an embodiment of the invention that makes use of the virtual grating technique. The drawing is showing the different system components; the system of this invention comprises an X-ray source (35) generating the X-ray beam (33), a presample mask of which the parallel grating bars (32) are shown, and a digital image detector (36). The X-ray is split into small beamlets by said parallel oriented grating bars which absorb part of the incident X-ray beam. The beamlets generate a radiation profile (34) on a limited portion of the detector (36) and the profile is measured by the set (30) of detector pixels (31) on which the beamlet falls. The use of the virtual grating technique omits the requirement of the presence of a physical detector grating, therefore in this diagram no physical detector grating is present.

Fig. 3 illustrates the effect of beam deflection on a beamlet caused by phase effects on an X-ray beam when passing through material. The left graph shows the situation of an undisturbed beamlet that is centred in the middle between two neighbouring detector pixels; the radiation detected by the two pixels is equally distributed. The graph on the right shows the situation where beam deflection occurred. The x-ray power incident on the left pixel (10) is reduced while the x-ray power incident on the right pixel (11) is increased. Thus the difference between the left (10) and right pixel (11) can be understood as a measure for the phase change.

Fig. 4 illustrates the effect of beam broadening caused by scatter. Compared to the undisturbed beamlet (in the left graph), the beamlet gets broader and lower when subjected to (scatter leaves the area under the curve unchanged - as there is no absorption). Due to beam broadening a part of the intensity is deflected to the neighbouring pixels. The pixel value of the left (10) and right pixel (11) goes down by the same amount, as can be seen in the graph on the right.

Fig. 5 illustrates the effect of the presence of a linear absorption gradient on a beamlet; for the phase image the net effect is that the centre of the measurement profile (12) is shifted (14) to the right (13) in this case (see left graph), which mimics the same effect as if there would have been a beam deflection. Consequently, the profile shifts off-centre to the right and the phase effect is overestimated in the phase image.

For the dark field image, due to the presence of a linear absorption gradient, the profile becomes more steep (13), consequently the scatter effect is underestimated.

Fig. 6 gives an overview of the acquisition sequence to acquire a PCI image at full (spatial) detector resolution in the case that a set of 4 pixels (30) are used to measure a beamlet profile on the digital imaging detector. The sequence of 4 x-ray absorption images is indicated in the horizontal direction of the drawing, and the individual X-ray absorption acquisitions are marked with I, II, III and IV. It is noted that the grating bars (32) of the pre-sample mask are positioned differently for each of these acquisitions; namely the positions of the grating bars ensure that the beam profile is centred every time on a different pixel pair, and shifts with a distance of a single pixel.

The drawing also gives a schematic overview of the different virtual grating positions that allow subsequent extraction of the images for the reconstruction of the PCI images. The drawings showing the different grating positions for the same x-ray absorption image are sorted vertically. The pixel modulations resulting in the respective different virtual grating positions are marked with capital letters A, B, C and D. An inactive pixel is herein indicated with a shaded background, whereas a used (active) pixel is indicated with a white background. As can be seen from the drawing, in the example with the set of 4 pixels being used to measure a beamlet profile, there are 4 different pixel modulations possible.

Fig. 7 illustrates (for the example with a set of 4 pixels) which profile data should be taken into consideration for the normalisation step of a certain exposure (acquired with a certain pre-sample mask position). As can be seen, the profile spanning the full 4 pixels of the pixel set can be used to normalize the profile data used to reconstruct the PCI images.

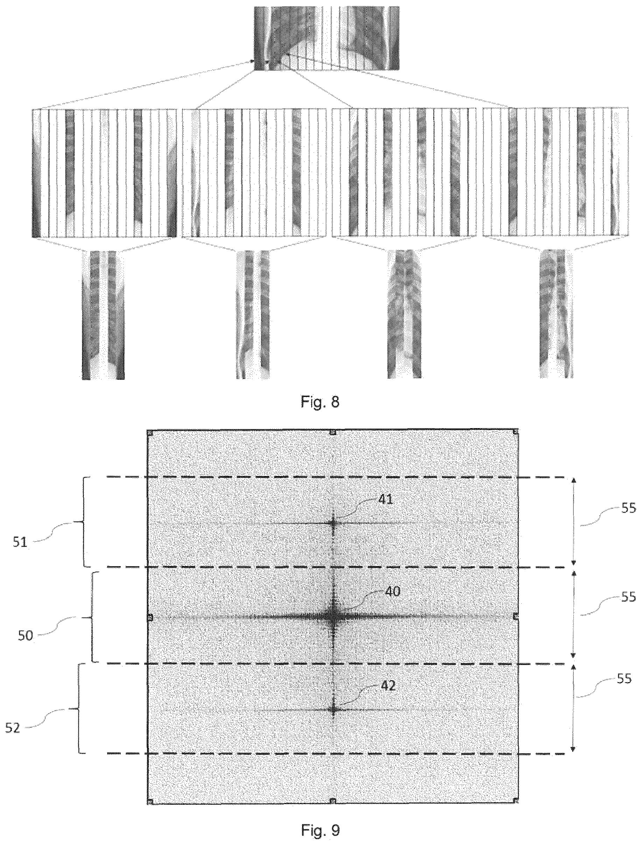

Fig. 8 illustrates the recombination of n corrected phase contrast images into one high-resolution PCI image. Each same pixel position values of said *n* corrected PCI images are collated into *n* consecutive pixels of the recombined high-resolution PCI image.

Fig. 9 represents a FFT (Fast Fourier Transformation) of a PCI acquisition (using a pre-sample mask) of a real object and shows the effect on the FFT magnitude. The changes in the 0th order are caused by absorption. The $0^{th}$ order peak is visible (40). The changes in the $1^{st}$ order magnitude are caused by beam broadening and the changes of the $1^{st}$ order phase represent the phase image. The $1^{st}$ order peak is visible as (41), (42) is the $-1^{st}$ order peak. The regions (50) and (51) in the transformed image represent respectively the $0^{th}$ and $1^{st}$ order Fourier Transformed portion of the image data that may be separated from the rest of the image and transformed back to obtain respectively the absorption image and the refracted image.

## Description of embodiments

The purpose of the invention herein disclosed is to reduce artefacts in the PCI image which are caused by steep absorption gradients in the imaged object without the need to acquire additional images for this purpose, which are normally required when using conventional PCI systems. The method disclosed can be applied to PCI systems that use a virtual detector grating.

This method to reduce the artefacts is based on a clever re-use of suitable acquired profile data that already was part of the acquisition data set made for the purpose of achieving full-resolution PCI images. The raw acquired absorption measurement data in combination with the application of a so-called virtual grating unexpectedly allows to extract the necessary information to correct the phase and dark field image data.

The method assumes that a number of pre-requisites regarding the geometry of the PCI system are met. The geometry of the PCI system is determined to a large extend by the geometry of the digital image detector which imposes limitations to the positioning and design of the other PCI system components. The pitch size of the detector $S_p$ determines the pitch size of the virtual analyser grating $p_{vg}$, which is chosen as a multiple of the detector pitch size:

$$p_{vg} = S_p . n; \ n \geq 4$$

This virtual analyser grating pitch $p_{vg}$ determines in its turn the pitch size of pre-sampling mask $p_{pm}$ that corresponds to the selected pitch of the virtual analyzer grating $p_{vg}$ divided by the magnification *M*:

$$p_{pm} = \frac{p_{vg}}{M}$$

Next, the aperture size $\alpha$ should be smaller than or equal to half the pitch of the pre-sampling mask in order to produce the desired profile shape to perform the desired profile measurements:

$$a \leq \frac{p_{pm}}{2}$$

A third consequence is that in order to compensate for the loss in spatial resolution, and thus in order to ensure that per digital image detector pixel a pixel of both the phase image and the dark field image can be reconstructed, the displacement of the pre-sampling mask $\Delta\xi$ must be equal to the pixel size of the detector $s_p$ divided by the magnification *M*:

$$\Delta\xi = \frac{s_p}{M}$$

The method relies on a sequence of steps which are initially common to the acquisition of PCI images on a conventional system; as a first step, a series of *n* conventional x-ray absorption images are acquired for which it is noted that each image is acquired with a slightly different pre-sample mask position. In other words the pre-sample mask is shifted in between the *n* different exposures so to cover the entire distance of the virtual analyser grating pitch $p_{vg}$ as the sum of the $n$ subsequent steps. The number $n$ may be arbitrarily to some extent, but determined by the number of full detector pixels that are comprised in a measured profile that allows us to reconstruct a single PCI image pixel. Therefore, the number of pixels $n$ should only cover the amount needed to record a meaningful intensity profile for a single beamlet. The number of detector pixels $n$ thus determines the width of the measured profile, but will then also require that the dimensions of the pre-sample mask and the magnification $M$ are adapted to this choice (as explained above). A higher value of $n$ allows a smoother profile measurement for one PCI image pixel, but at the same time reduces the spatial resolution of the image, which can however be compensated by acquiring $n$ acquisitions (the higher the value of $n$, the more acquisitions are required to achieve the same spatial resolution). In a preferred embodiment the number $n$ equals 4, which can be considered to be the absolute minimum.

In the further explanation, including the drawings to which reference is made, the number $n$ is chosen to equal 4. However, this is not a limitation for the applicability of this invention.

The above series of $n$ acquired x-ray absorption images are represented in Fig. 6 as images I, II, III and IV. For each of these images, it is shown in the drawing that pre-sample mask grating bars (32) are shifted as indicated by the arrows for each of the acquisitions I to IV. Consequently, the radiation profiles also shift to expose a different set of detector pixels (30). Each set of said detector pixels (30) will allow to reconstruct one pixel in the phase and dark field image.

In conventional PCI imaging, a portion of the pixels of the digital image detector would have to be blocked (by the grating bars of the analyser grating) in order to obtain the necessary image data to reconstruct the phase and dark filed images. Typically the re-calculation of the phase image is based on the difference of two images recorded at different positions of the analyser grating. The scatter image typically uses the sum of two normalized images. This means that multiple images need to be acquired for the same pre-sample mask position in order to be able to reconstruct a single phase or dark field image.

Consequently, in order to achieve the full image detector resolution in the PCI images, a multiple of $n$ more conventional PCI acquisitions would be required. For each pre-sample mask position ($n$ in total) a multiple of radiation profile data would have to be acquired shifting the analyser grating to different positions in order to obtain the desired profile data.

In a preferred embodiment of the invention, the PCI image profile data are acquired more efficiently using the virtual grating technique. Since no physical analyser grating (or detector grating) is used, there is no need to make real acquisitions for each physical analyser grating movement. Instead, the same digital image that is acquired while being subjected to a certain pre-sample mask position, is processed as such that selected lines (or other configurations) of pixels (which take the place of the position of the conventional grating bars) are omitted or not taken into consideration for reconstruction. As such, a so-called virtual mask is laid over the digital image matrix thereby determining the locations of the image which are considered to be taken into account (or are considered "switched on").

Fig. 6 illustrates the pixel modulation that is applied by the virtual grating on each sequence of 4 detector pixels (30). The different pixel modulations (A, B, C and D) are shown for each of the $n$ acquired x-ray absorption images (I, II, III and IV) in the respective columns. The active/inactive pixels are shown with a different shading color. One of the 4 modulations is not used for image reconstruction, as this sequence does not capture any of the pixel values in the center of the radiation profile (this corresponds to a grating bar blocking the central part of the profile). These unused modulations are crossed-out in Fig. 6.

In case that the phase and dark field image would be reconstructed based entirely on the above pixel modulated images (as to mimic the physical grating), the results would be uncorrected for gradient artefacts.

Alternatively, rather than to perform $n$ consecutive pixel modulations in real-space on each of the $n$ images acquired using different pre-sample mask positions in order to extract the measurement profiles for reconstructing the PCI images as explained above, it is possible to perform the same operation in the Fourier domain after transformation of the n images to the Fourier space. The basis for this is the fact that a periodic image (such as an intensity modulated image by a grating) produces a Fourier transform with a 0-order (that is related to the constant part of the image), a 1st order related to the main frequency of the modulation (i.e. the frequency of the pre-sample mask) and higher orders (harmonics) which are related to multiples of the frequency of the 1st order. Refraction and scatter influence the periodic part of the intensity profile and thus cause changes in the 1st order peak of the Fourier transformation. The complex 2D Fourier transform thus has a characteristic look as shown in Fig. 9 on which identifiable peaks are visible.

It suffices to filter out the desired portion of this complex 2D (two dimensional) Fourier transform representing the 1st order signal in order to perform the same operation as the virtual grating does in real space. The 2D Fourier transformed image shows 3 identifiable regions (or sub-arrays) centered on 3 clearly identifiable peaks. A first region (50) is centered on the 0th order peak (40) and the region height is defined by the distance (55) (expressed in number of pixels) between the 0th and 1st order peak. The width of this region is defined by the width of the raw image as we use a 1D grating. A second region is centered at the 1st order peak (51) and again the region height is defined by the distance (55) between the 0th and 1st order peak. These identified regions are separated and back transformed (using a complex inverse Fourier transform) and result in 3 images:

1- the absorption image is obtained through back transformation of the magnitude of the region (50) centered on the 0th order peak (40),

2- the refraction image is obtained through back transformation from the phase data of the back transformed region (51) centered on the 1st order peak (41) (which is in fact an angle in the complex plane),

3- the scatter image is obtained from the magnitude of the back transformation of the region (51) centered on the 1st order peak (41) divided by the absorption image.

Still, the obtained images would be uncorrected for gradient artefacts. As explained before, in order to correct for this type of artefact, the conventional approach (using a physical analyser grating) would be to acquire additional profile data that would allow to normalize a radiation profile with profiles centered on its closest neighbouring pixels. The normalization would be done in the case of our preferred embodiment of 4 pixel-sized sets with the at least 2 neighbouring (left and right) profiles.

However, we surprisingly found that no additional image acquisitions are required when applying the virtual grating technique; the required normalization profiles are already available in already acquired absorption images. Selecting the correct image data and applying the correct profile data of said image data for normalizing the corresponding PCI image allows to perform the correction without the need for additional acquisitions.

Fig. 7 illustrates for our example which profile data should be taken into consideration for the normalisation step of certain a exposure (I, II, III or IV). As can be seen, the profile spanning the full 4 pixels of the pixel set can be used to normalize the profile data used to reconstruct the PCI images.

In a preferred embodiment, the normalization of a certain measured profile is performed by comparing the maximum of this considered measurement profile with the maxima of the profiles centred on its closest neighbouring pixels. A deviation between the considered maximum and the respective neighbouring maxima vouches for the presence of an absorption gradient. The maximum of the measured profile in question can then be adjusted to better align to the values of its neighbours. This alignment is achieved through normalisation, or through any other means of adjustment.

The recalculation of the phase $ph$ then typically consists of two terms: an uncorrected phase $ph_{nc}$ and a correction term $c$. The terms are functions of the pixel values $I_1$ & $I_2$, representing the 2 most central pixels of the set of 4 pixels (refer to Fig. 7).

$$ph = ph_{nc}\left(I_1|_{\xi=0};\ I_2|_{\xi=0}\right) - c\left(I_1|_{\xi=-\Delta\xi};\ I_2|_{\xi=-\Delta\xi};\ I_1|_{\xi=+\Delta\xi};\ I_2|_{\xi=+\Delta\xi}\right)$$

The correction factor c depends on the exact geometry of the setup and to some extend also on the absorption and transmission of the pre-sample mask

The method described above can also be applied to correct scatter image re-calculation. The approach is similar. The main difference is the calculation of the correction factor c. It may also be advantageous to use other pixel values than those of the centred pixels as the absorption gradient impacts the calculation of the scatter image differently (see fig. 5). For someone skilled in the art it is obvious that the absorption gradient correction can be used to correct phase images and to correct scatter images in an almost similar way as the effect on the shape of the intensity profile of a beamlet is similar in both cases. I.e. an absorption gradient shifts and narrows the profile of a beamlet - for phase correction the shift has to be calculated for scatter correction the narrowing has to be calculated.

**Claims**

1. Computer-implemented method for generating a high-resolution Phase-Contrast X-ray image corrected for absorption gradient artefacts in a phase image or a dark field image on a Phase-Contrast X-ray imaging system, comprising the steps of:

   - acquiring a series of $n \geq 4$ x-ray absorption images on an x-ray detector (36), acquiring said x-ray absorption images at $n$ different positions $\xi_{i=1 \to n}$ of a pre-sample mask (32) in said Phase-Contrast X-ray imaging system, wherein

   $$\xi_{i=1 \to n} = i . \frac{s_p}{M}$$

   , $s_p$ is the pixel size of said x-ray detector (36), and M is the magnification factor of the Phase-Contrast X-ray imaging system, said pre-sample mask having a pitch size $p_{pm}$ that corresponds to the selected pitch of the virtual analyzer grating $p_{vg}$ divided by the magnification $M$:

$$p_{pm} = \frac{p_{vg}}{M}$$

, the pitch size of the virtual analyser grating $p_{vg}$ is determined by the pitch size of the detector $S_p$, wherein:

$$p_{vg} = S_p . n; \ n \geq 4$$

- computing $n$ uncorrected Phase-Contrast X-ray images for each of said $n$ x-ray absorption images, wherein said Phase-Contrast X-ray image is a phase image or a dark field image, each uncorrected Phase-Contrast X-ray image being calculated from $n$ different sets of the total number of pixels detected by said x-ray detector, said $n$ sets being obtained by virtually overlaying a pixel matrix read out of the exposed detector by a virtual mask having on/off locations and by using pixels covered by 'on' locations of the virtual mask to populate a first set and shifting said virtual mask relative to the pixel matrix to obtain further sets;
- calculating $n$ corrected Phase-Contrast X-ray images wherein said Phase-Contrast X-ray image is a phase image or a dark field image, by correcting phase profile measurements from said phase image and correcting scatter profile measurements from said dark field image at each detector position of said $n$ uncorrected Phase-Contrast X-ray images by normalizing said uncorrected profile measurements with at least 2 acquired profile measurements from directly neighbouring pre-sample mask positions and corresponding detector pixels, said profile measurements from directly neighbouring pre-sample mask positions obtainable from the corresponding neighbouring image from the series of $n$ x-ray absorption images,
- recombining said $n$ corrected Phase-Contrast X-ray images into respectively a high-resolution phase image and a high-resolution dark field image, wherein each same pixel position values of said $n$ corrected Phase-Contrast X-ray images are collated into $n$ consecutive pixels of the respective recombined high-resolution phase image and dark field image.

2. The method according to Claim 1, wherein the value of $n$ is 4, 6 or 8.

3. The method according to Claim 1, where the normalization of the uncorrected profile measurements is done by comparing direct pixel values of the X-ray detector.

4. The method according to Claim 1, where the normalization of the uncorrected profile measurements is done by comparing the logarithms of pixel values of the X-ray detector.

5. Method according to Claim 1, where computing $n$ uncorrected Phase-Contrast X-ray images for each of said $n$ x-ray absorption images, wherein said Phase-Contrast X-ray image is a phase image or a dark field image, is performed by transformation of each of said $n$ x-ray absorption images to the Fourier space, followed by selecting a subset of the Fourier transformed image, and by performing a back transformation of said selected subset.

6. The method according to Claim 5 wherein the sub-set of the Fourier transformed image is defined as a rectangular region centered on the 0th or the 1st order peak of said image, and wherein a region height is defined by the distance between the $0^{th}$ and $1^{st}$ order peak, and a region width is defined by the width of the image.

7. The method according to Claim 5 or 6, wherein a corrected Phase-Contrast X-ray image is obtained through normalization of said uncorrected Phase-Contrast X-ray image followed by the calculation from a Fourier transformation of the detector data, selecting a sub-set of the Fourier transformed image and a back-transformation of said selected sub-set.

8. The method according to Claim 5, where the uncorrected profile calculation is based on a Fourier transformation and correction is applied after the transformations by normalization using a second and third Fourier transformation and back-transformation of sub-regions to acquire the correction data.

**Patentansprüche**

1. Computerimplementiertes Verfahren zur Erzeugung eines hochauflösenden Phasenkontraströntgenbildes mit korrigierten Absorptionsgradientartefakten in einem Phasenbild oder Dunkelfeldbild auf einem Phasenkontraströntgenbildgebungssystem, umfassend die folgenden Schritte:

   - Erfassung einer Serie von $n \geq 4$ Röntgenabsorptionsbildern auf einem Röntgendetektor (36), Erfassung dieser Röntgenabsorptionsbilder an $n$ unterschiedlichen Positionen $\xi_{i=1 \to n}$ einer Presample-Maske (32) im Phasenkontraströntgenbildgebungssystem, wobei

   $$\xi_{i=1 \to n} = i.\frac{S_p}{M}$$

   wobei $S_p$ die Pixelgröße des Röntgendetektors (36) ist und M der Vergrößerungsfaktor des Phasenkontraströntgenbildgebungssystems ist,

   wobei die Presample-Maske eine Pitchgröße $p_{pm}$ aufweist, die dem gewählten Pitch des Gitters eines virtuellen Analysators $p_{vg}$, geteilt durch den Vergrößerungsfaktor M, entspricht:

   $$p_{pm} = \frac{p_{vg}}{M}$$

   wobei die Pitchgröße des Gitters des virtuellen Analysators $p_{vg}$ durch die Pitchgröße des Detektors $S_p$ bestimmt wird, wobei:

   $$p_{vg} = S_p.\mathrm{n}; \mathrm{n} \geq 4$$

   - Berechnung $n$ nicht-korrigierter Phasenkontraströntgenbilder für jedes der $n$ Röntgenabsorptionsbilder, wobei das Phasenkontraströntgenbild ein Phasenbild oder ein Dunkelfeldbild ist, wobei jedes nicht-korrigierte Phasenkontraströntgenbild aus $n$ unterschiedlichen Sätzen der Gesamtanzahl von durch den Röntgendetektor detektierten Pixeln berechnet wird, wobei die $n$ Sätze erhalten werden, indem die aus dem belichteten Detektor ausgelesene Pixelmatrix virtuell mit einer virtuellen Maske mit On/Off-Stellen überlagert wird und durch "On"-Stellen der virtuellen Maske abgedeckte Pixel der virtuellen Maske verwendet werden, um einen ersten Satz zu füllen, und die virtuelle Maske relativ zur Pixelmatrix verschoben wird, um weitere Sätze zu erhalten,
   - Berechnung $n$ korrigierter Phasenkontraströntgenbilder, wobei das Phasenkontraströntgenbild ein Phasenbild oder ein Dunkelfeldbild ist, durch Korrektur von Phasenprofilmessungen aus dem Phasenbild und durch Korrektur von Streuprofilmessungen aus dem Dunkelfeldbild an jeder Detektorposition der $n$ nicht-korrigierten Phasenkontraströntgenbilder, indem die nicht-korrigierten Profilmessungen mit mindestens 2 erfassten Profilmessungen aus direkt benachbarten Positionen der Presample-Maske und entsprechenden Detektorpixeln normalisiert werden, wobei die Profilmessungen aus direkt benachbarten Positionen der Presample-Maske aus dem entsprechenden benachbarten Bild der Serie von $n$ Röntgenabsorptionsbildern erhalten werden können,
   - Rekombinieren der $n$ korrigierten Phasenkontraströntgenbilder zu einem hochauflösenden Phasenbild bzw. einem hochauflösenden Dunkelfeldbild, wobei alle gleichen Pixelpositionswerte der $n$ korrigierten Phasenkontraströntgenbilder zu $n$ aufeinanderfolgenden Pixeln des jeweiligen rekombinierten hochauflösenden Phasenbildes und hochauflösenden Dunkelfeldbildes gesammelt werden.

2. Das Verfahren nach Anspruch 1, wobei der $n$-Wert 4, 6 oder 8 ist.

3. Das Verfahren nach Anspruch 1, wobei die Normalisierung der nicht-korrigierten Profilmessungen durch Vergleich direkter Pixelwerte des Röntgendetektors erfolgt.

4. Das Verfahren nach Anspruch 1, wobei die Normalisierung der nicht-korrigierten Profilmessungen durch Vergleich der Logarithmen von Pixelwerten des Röntgendetektors erfolgt.

5. Das Verfahren nach Anspruch 1, wobei die Berechnung n nicht-korrigierter Phasenkontraströntgenbilder für jedes der n Röntgenabsorptionsbilder, wobei das Phasenkontraströntgenbild ein Phasenbild oder Dunkelfeldbild ist, durch Transformation jedes der n Röntgenabsorptionsbilder zum Fourier-Raum und anschließendes Wählen eines Teilsatzes des Fourier-transformierten Bildes und Durchführung einer Rücktransformation des gewählten Teilsatzes erfolgt.

6. Das Verfahren nach Anspruch 5, wobei der Teilsatz des Fourier-transformierten Bildes als auf dem Peak nullter oder erster Ordnung des Bildes zentrierte rechteckige Region definiert wird und wobei die Regionhöhe durch den Abstand zwischen dem Peak nullter Ordnung und dem Peak erster Ordnung definiert wird und die Regionbreite durch die Breite des Bildes definiert wird.

7. Das Verfahren nach Anspruch 5 oder 6, wobei ein korrigiertes Phasenkontraströntgenbild durch Normalisierung des nicht-korrigierten Phasenkontraströntgenbildes und anschließend die Berechnung aus einer Fourier-Transformation der Detektordaten, Wahl eines Teilsatzes des Fourier-transformierten Bildes und Rücktransformation des gewählten Teilsatzes erhalten wird.

8. Das Verfahren nach Anspruch 5, wobei die nicht-korrigierte Profilberechnung auf einer Fourier-Transformation basiert und zum Erhalt der Korrekturdaten nach den Transformationen durch Normalisierung mittels einer zweiten und dritten Fourier-Transformation und Rücktransformation von Teilregionen eine Korrektur angewandt wird.

**Revendications**

1. Procédé mis en œuvre par ordinateur servant à la génération d'une image radiographique haute résolution à contraste de phase qui est corrigée pour les artéfacts à gradient d'absorption dans une image de phase ou une image en champ sombre sur un système d'imagerie radiographique à contraste de phase, comprenant les étapes consistant à:

- acquérir une série de n ≥ 4 images d'absorption de rayonnement X sur un détecteur de rayons X (36), acquérir lesdites images d'absorption de rayonnement X à n positions $\xi_{i=1\rightarrow n}$ différentes d'un masque de pré-échantillon (32) dans ledit système d'imagerie radiographique à contraste de phase, où

$$\xi_{i=1\rightarrow n} = i.\frac{S_p}{M}$$

où $S_p$ est la taille de pixel dudit détecteur de rayons X (36) et M est le facteur de grandissement du système d'imagerie radiographique à contraste de phase,

ledit masque de pré-échantillon ayant une taille de pitch $p_{pm}$ qui correspond au pitch sélectionné de la grille d'un analyseur virtuel $p_{vg}$ divisé par le facteur de grandissement M:

$$p_{pm} = \frac{p_{vg}}{M}$$

la taille de pitch de la grille de l'analyseur virtuel $p_{vg}$ étant déterminée par la taille de pitch du détecteur $S_p$, où:

$$p_{vg} = S_p.\text{n}; \text{n} \geq 4$$

- calculer *n* Images radiographiques à contraste de phase non corrigées pour chacune desdites n images d'absorption de rayonnement X, où ladite image radiographique à contraste de phase est une image de phase ou une image en champ sombre, chaque image radiographique à contraste de phase non corrigée étant calculée à partir de *n* ensembles différents du nombre total de pixels détectés par ledit détecteur de rayons X, lesdits *n* ensembles étant obtenus en superposant virtuellement la matrice de pixels lue à partir du détecteur exposé à l'aide d'un masque virtuel ayant des positions "on/off" et en utilisant des pixels recouverts par des positions "on" du masque virtuel afin de remplir un premier ensemble et en déplaçant ledit masque virtuel par rapport à la matrice de pixels afin d'obtenir des ensembles supplémentaires,
- calculer n images radiographiques à contraste de phase corrigées, où ladite image radiographique à contraste de phase est une image de phase ou une image en champ sombre, en corrigeant des mesurages de profil de phase à partir de ladite image de phase et en corrigeant des mesurages de profil de diffusion à partir de ladite image en champ sombre à chaque position de détecteur desdites n images radiographiques à contraste de phase non corrigées en normalisant lesdits mesurages de profil non corrigés étant normalisés avec au moins 2 mesurages de profil acquis à partir de positions directement voisines du masque de pré-échantillon et de pixels de détecteur correspondants, lesdits mesurages de profil obtenus à partir de positions directement voisines du masque de pré-échantillon pouvant être obtenus à partir de l'image voisine correspondante de la série de *n* images d'absorption de rayonnement,

- recombiner lesdites *n* images radiographiques à contraste de phase corrigées en respectivement une image de phase haute résolution et une image en champ sombre haute résolution, toutes les valeurs de position de pixel identiques desdites *n* images radiographiques à contraste de phase corrigées étant rassemblées en *n* pixels successifs de l'image de phase haute résolution recombinée et de l'image en champ sombre haute résolution recombinée.

2. Procédé selon la revendication 1, **caractérisé en ce que** la valeur *n* est égale à 4, 6 ou 8.

3. Procédé selon la revendication 1, **caractérisé en ce que** la normalisation des mesurages de profil non corrigés s'effectue en comparant des valeurs de pixel directes du détecteur de rayons X.

4. Procédé selon la revendication 1, **caractérisé en ce que** la normalisation des mesurages de profil non corrigés s'effectue en comparant les logarithmes de valeurs de pixel du détecteur de rayons X.

5. Procédé selon la revendication 1, **caractérisé en ce que** le calcul de *n* images radiographiques à contraste de phase non corrigées pour chacune desdites *n* images d'absorption de rayonnement X, ladite image radiographique à contraste de phase étant une image de phase ou une image en champ sombre, s'effectue en exécutant une transformation de chacune desdites *n* images d'absorption de rayonnement X vers l'espace de Fourier et en sélectionnant ensuite un sous-ensemble de l'image soumise à la transformation de Fourier et en effectuant une rétrotransformation dudit sous-ensemble sélectionné.

6. Procédé selon la revendication 5, **caractérisé en ce que** le sous-ensemble de l'image soumise à une transformation de Fourier est défini comme une région rectangulaire centrée sur le pic d'ordre zéro ou le pic de premier ordre de ladite image et que la hauteur de région est définie par la distance entre le pic d'ordre zéro et le pic de premier ordre et que la largeur de région est définie par la largeur de l'image.

7. Procédé selon la revendication 5 ou 6, **caractérisé en ce qu'**une image radiographique à contraste de phase corrigée est obtenue en normalisant ladite image radiographique à contraste de phase non corrigée et en effectuant ensuite le calcul à partir d'une transformation de Fourier des données de détecteur, en sélectionnant un sous-ensemble de l'image soumise à une transformation de Fourier et en effectuant une rétrotransformation dudit sous-ensemble sélectionné.

8. Procédé selon la revendication 5, **caractérisé en ce que** le calcul de profil non corrigé est basé sur une transformation de Fourier et qu'après les transformations il est effectué une correction par normalisation au moyen d'une première et deuxième transformation de Fourier et une rétrotransformation de régions partielles afin d'obtenir les données de correction.

intensity profile & pixel value
for a single beamlet

displacement of coded aperture

32   38        36        38

37        36

Fig. 1

Fig. 2

Fig. 3

Fig. 4

16

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 17181154
- US 2017307549 A1
- WO 2017015381 A1
- US 2017213364 A
- EP 17181154 A

**Non-patent literature cited in the description**

- Single grating method for low dose 1-D and 2-D phase contrast X-ray imaging. **KREJCI F et al.** JOURNAL OF INSTRUMENTATION. INSTITUTE OF PHYSICS PUBLISHING, vol. 6
- **FABIO DE MARCO et al.** Analysis and correction of bias induced by phase stepping jitter in grating-based X-ray phase-contrast imaging. *OPTICS EXPRESS,* 03 May 2018, vol. 26 (10